(19) **Europäisches Patentamt**

**European Patent Office**

**Office européen des brevets**

(11) **EP 1 381 400 B1**

(12) **EUROPEAN PATENT SPECIFICATION**

(45) Date of publication and mention
of the grant of the patent:
**03.11.2004 Bulletin 2004/45**

(51) Int Cl.$^7$: **A61L 2/14**, A61L 2/28

(86) International application number:
**PCT/GB2002/001907**

(21) Application number: **02718391.2**

(22) Date of filing: **25.04.2002**

(87) International publication number:
**WO 2002/087637 (07.11.2002 Gazette 2002/45)**

(54) **STERILIZATION METHOD**

STERILISIERVERFAHREN

PROCEDE DE STERILISATION

(84) Designated Contracting States:
**DE FR GB**

(30) Priority: **25.04.2001 GB 0110191**

(43) Date of publication of application:
**21.01.2004 Bulletin 2004/04**

(73) Proprietor: **Oxford Instruments Plasma
Technology Limited
Witney, Oxfordshire OX8 1TL (GB)**

(72) Inventor: **FIELD, John
Limpley Stoke, Bath BA2 7GR (GB)**

(74) Representative:
**Skone James, Robert Edmund et al
GILL JENNINGS & EVERY,
Broadgate House,
7 Eldon Street
London EC2M 7LH (GB)**

(56) References cited:
**EP-A- 0 596 861       WO-A-00/72889
WO-A-01/13964        WO-A-95/29709
US-A- 5 920 799**

• **THE T C ET AL: "Microwave plasmas for
low-temperature dry sterilization"
BIOMATERIALS, ELSEVIER SCIENCE
PUBLISHERS BV., BARKING, GB, vol. 17, no. 13,
1996, pages 1273-1277, XP004032641 ISSN:
0142-9612**
• **DATABASE WPI Section Ch, Week 199937
Derwent Publications Ltd., London, GB; Class
D22, AN 1999-437607 XP002183746 & JP 11
178904 A (HOGY MEDICAL KK), 6 July 1999
(1999-07-06)**

EP 1 381 400 B1

**Description**

The invention relates to a method of sterilizing an object contaminated with organic material.

Conventionally, medical instruments and the like need to be carefully sterilized before use in medical procedures. There are many techniques for achieving sterilization such as heating to a high temperature, or the use of antimicrobial agent such as hydrogen peroxide or ethylene oxide, and the like. Recently, with the growth in concern about the transfer of CJD and similar diseases by organic contamination such as prions, conventional sterilization techniques have been considered unsafe and disposable instruments have been developed for certain medical procedures, which are destroyed after use. Although this is an acceptable approach, problems arise with certain instruments which are often complex and too expensive to throw away after a single use.

In accordance with the present invention, a method of sterilizing an object carrying organic material comprises exposing the object to a plasma of ionised gas and simultaneously exposing a monitoring object to the same plasma, the monitoring object comprising a substrate carrying a substance,

whereby the organic material on the object and the substance on the substrate each react with the plasma to generate one or more volatile products such that the reaction causes a visible change in the monitoring object by the removal of the substance from the substrate;

removing the product(s); and

monitoring the monitoring object to determine whether exposure to the plasma has occurred.

We have realized that the use of a plasma to achieve sterilization may overcome the problems outlined above. The use of plasmas is well known in the area of semiconductor fabrication. We have realized that many processes are based on organic compounds and thus surprisingly this use of a plasma could be adapted for szerilizacion. In che context of the present invention, the term "sterilization" is intended to mean a process to effect a clean ("sterile") environment by the destruction and/or removal of organic material in any form. In particular, the organic material is intended to include "non-living" entities such as prions.

An important application of the invention is therefore where the organic material comprises prions but it could also be used with other organic material such as "living" material, as with other sterilization techniques.

The plasma typically comprises ionized oxygen but other gases such as the Fluorine based gases, for example carbon tetrafluoride, could be used or added. Other examples of fluorine based gases are $CHF_3$ and $C_2F_6$. Other gases commonly used for the etching of organic materials are: $CH_4$, $BCl_3$, Ar and $N_2$.

Occasionally, it is has been found in the semiconductor industry that the equipment fails to generate a plasma and the operator is unaware of this when he unloads the object and passes it on to the next manufacturing stage. This has been widely recognized in the semiconductor industry and methods and checks have been developed to deal with it. These typically involve the use of extra instruments to check that the plasma has been ignited. For example, a photodetector is often used to detect the light from the discharge. However, if a multiple failure is considered then it is possible to imagine a set of circumstances whereby the machine still appears to have operated successfully but in fact has not. Although the probability of this is so low as to be acceptable in the semiconductor industry, it would not be acceptable in sterilization procedures.

The method further comprises simultaneously exposing a monitoring object to the same plasma, the monitoring object comprising a substrate carrying a substance which reacts with the plasma to undergo a visible change.

In this way, the operator can check the monitoring object to see whether it has reacted with the plasma and if it has, this will confirm that the plasma has been generated and thus the object has been sterilized.

Typically, the substance reacts with the plasma to generate one or more volatile products and so may comprise a photoresist or the like. Conveniently the substance is opaque, the substrate carrying indicia covered by the substance and which are revealed following reaction of the substance with the plasma.

Any conventional plasma generating apparatus could be used to implement the method, a particularly suitable apparatus being a Barrel Reactor.

An example of apparatus for carrying out a method according to the present invention will now be described with reference to the accompanying drawings, in which:-

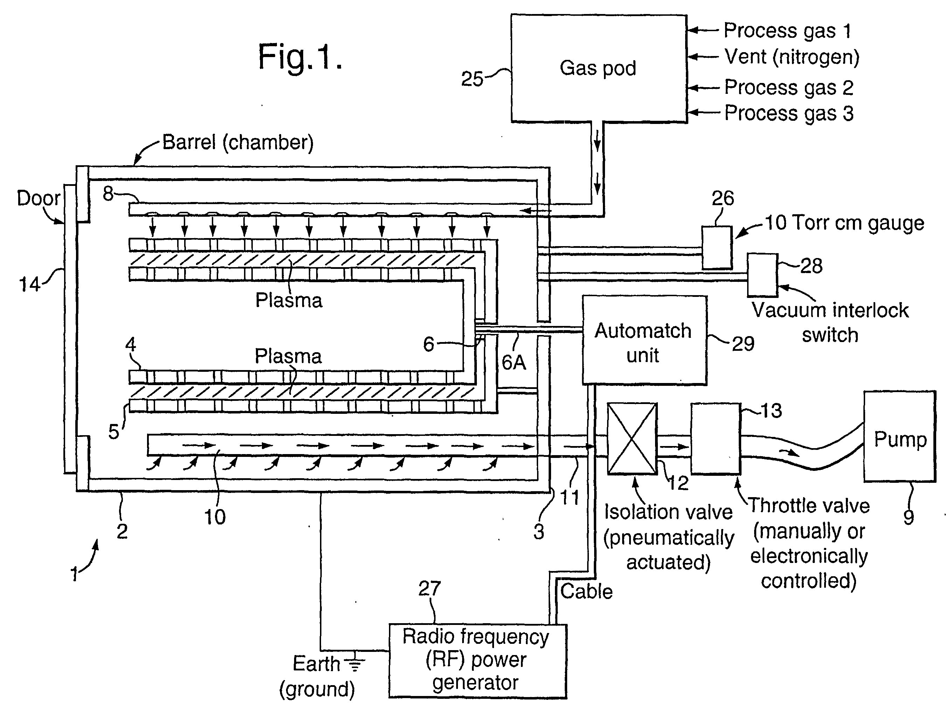

Figure 1 is a schematic block diagram of the apparatus;
Figure 2 is a cross-section through a monitoring object;
Figure 3 is a plan of the monitoring object shown in Figure 2 with the photoresist layer removed; and,
Figure 4 is a flow diagram of an example method using the apparatus.

The drawing illustrates a barrel reactor sold by Oxford Instruments Plasma Technology Limited under the name Plasmalab™. The reactor comprises a barrel chamber 1 consisting of a horizontal aluminium cylinder 2 with a bolted or welded back-plate 3.

A pair of electrodes 4,5 consisting of two aluminium cages are provided, one inside the other. The outer electrode 5 is bolted to the barrel 2, and the inner 4 is secured to the outer 5, but they are insulated from one another by means of ceramic spacers 6. A feedthrough 6A carries RF power input.

The gas inlet to the barrel chamber 1 is via a tube 8 which runs parallel with the top of the chamber and above the outer electrode 5.

A pump 9 is connected to a large diameter tube 10 running along parallel with the chamber beneath the outer cage 5, leaving the chamber via a large diameter extension tube 1. This tube is O-ring sealed to the backplate 3 and terminates in a high vacuum valve 12 which is connected to the pump 9 via a throttle valve 13.

The chamber has a front door 14 containing a Pyrex glass viewport and seals into an O-ring surrounding the door aperture by means of adjustable spring hinges (not shown), which allow a parallel closing action.

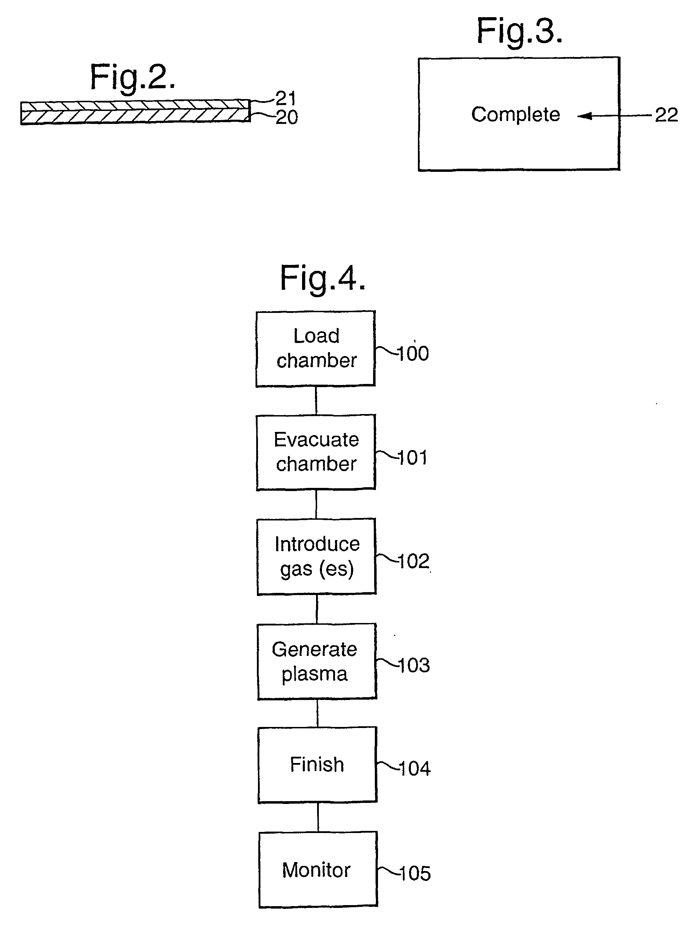

Referring to Figure 4, in operation at step 100, an instrument to be sterilized is placed in the chamber 2 supported by the inner cage 4. In addition, a monitoring object is positioned in the chamber.

An example of a monitoring object is shown in Figures 2 and 3. The object comprises a base 20 on which has been deposited a photoresist 21. The base 20 carries a printed message which can be seen in Figure 3 at 22. This message is obscured initially by the photoresist 21.

The chamber door 14 is then closed and the isolation valve 12 between the chamber and the pump (which normally runs continuously) is opened. The air is then pumped out of the chamber to create a very "high" vacuum at step 101.

The process gases are then introduced via a gas pod 25 into the chamber via two tubes 8 (only one shown) which run parallel to the top of the chamber (step 102). The gases are controlled by electrically operated valves and mass flow controllers (not shown). Typically the pressure is about 1 Torr, roughly one thousandth of an atmosphere and this is monitored by a gauge 26.

A Radio Frequency (RF) generator 27 is then switched on at step 103. This is connected to the electrodes 4,5 in the chamber via an Automatch unit 29.

A vacuum interlock switch 28 ensures that neither the RF nor the process gases are enabled before the chamber pressure is below a set level.

The processing pressure in the chamber is controlled either manually by the throttle valve 13 located between the pump 9 and the isolation valve 12, or by a fully automatic adaptive pressure controller which provides separate pressure and gas flow controls.

Typically, the pump 9 is of the rotary vane type. In these, an oil sealed eccentrically mounted vane traps the gas as it rotates and sweeps it to the outlet port.

The RF generator power transforms the gases into a plasma in the space between the two electrodes 4,5.

The impedance (a form of electrical resistance present in circuits having oscillating current) of the plasma varies from one process to another and also during a process. Since the RF generator 27 is designed to operate into a fixed load or impedance, an element whose impedance automatically adjusts to suit the varying plasma load must be used. The Automatch Unit 29, which is a motorised network of capacitors and inductors, performs this function.

In more detail, to create a plasma, energy is imparted to a stable gas to excite the free electrons causing collisions thereby creating ions and radicals. For example, an electron colliding with an oxygen molecule can produce radicals as follows:

$$e + O_2 \Rightarrow O + O + e$$

Or, rather then separating the stable O molecule, an electron may be dislodged yielding:

$$e + O_2 \Rightarrow O_{2+} + 2e$$

The process converts relatively unreactive gas molecules into very reactive radicals and ions.

A typical reaction using an oxygen based plasma in the context of etching an organic material such as polyethelene $(CH_2)_n$, has the form:

$$(CH_2)_n + 2O \Rightarrow CO + H_2O$$

Alternatively, with a fluorine based gas such as carbon tetrafluoride, a reaction of the following form will take place:

$$\{CH_2)_n + 6F \Rightarrow CF_4 + 2HF$$

Both of these reactions result in the generation of volatile products, these can be pumped away and rendered harmless by conventional exhaust treatment methods.

During this process at step 103 when the plasma is present within the chamber 2, any organic material present upon the instrument in question, along with the photoresist layer upon the monitoring object, reacts with the plasma. Reactions of the kind mentioned above break down this organic material and generate the resultant volatile products which are then removed from the chamber as a result of the gas flow.

Any microorganisms within the chamber are therefore killed by the plasma. In addition and in contrast to known methods, the present invention also produces a sterile environment by breaking down and removing other organic materials such as prions. As prions are relatively simple organic structures rather than living organisms, they are consequently more difficult to render harmless. The break down of the organic material and its removal in the form of volatile products, cleans the objects within the chamber since the organic material is physically removed.

The present invention advantageously provides an environment in which the desired plasma conditions can be generated and maintained indefinitely. This allows organic materials to be subjected to a prolonged attack which is capable of destroying and removing even the most resistant prions.

During this process it is important to generate optimized plasma conditions for the destruction and removal of organic matter, since the behaviour of a plasma is strongly dependent upon such conditions. In fact, non-ideal conditions may serve to actually "bake" the organic material onto the object in question. The controlled introduction and removal of the gases from the chamber ensures that a constant and stable environment is produced which does not vary as a function of time. Such an environment is ideal for generating optimized plasma conditions. Typical processing times of the order of tens of minutes are often needed to ensure the complete removal of prion materials and these processing times can be achieved using the present method.

Although a gas flow is present during the process, uniformity of the gas composition within the chamber can be achieved by careful design.

Once the process of removing the organic material has been completed, the generation of the plasma is halted at step 104. The chamber is then returned to atmospheric pressure. At step 105, the monitoring object is examined either visually or with the aid of appropriate apparatus to determine whether the photoresist layer 21 has been removed and therefore whether the plasma process has been effective. It should also be noted that the monitoring step 105 could be performed in situ whilst the instrument and monitoring object are present within the chamber. This could be achieved by the use of the glass view port within the front door 14.

It is envisaged that the present method may be used in combination with or as a replacement for conventional sterilization methods.

## Claims

1. A method of sterilizing an object carrying organic material, the method comprising:-

   exposing the object to a plasma of ionised gas and simultaneously exposing a monitoring object (20) to the same plasma, the monitoring object comprising a substrate carrying a substance,
   whereby the organic material on the object and the substance on the substrate each react with the plasma to generate one or more volatile products such that the reaction causes a visible change in the monitoring object by the removal of the substance from the substrate;
   removing the product(s); and
   monitoring the monitoring object to determine whether exposure to the plasma has occurred.

2. A method according to claim 1, wherein the organic material comprises prions.

3. A method according to claim 1 or claim 2, wherein the plasma is an oxygen or fluorine gas based plasma.

4. A method according to any of claims 1 to 3, wherein the substance comprises a photoresist (21).

5. A method according to any of claims 1 to 4, wherein the substance is opaque, the substrate carrying indicia covered by the substrate and which are revealed following reaction of the substance with the plasma.

6. A method according to any of the preceding claims, wherein the plasma is generated within a chamber (2) and, during exposure of the object to the plasma, one or more gases are supplied to and removed from the chamber such that a substantially constant pressure is maintained within the chamber.

7. A method according to claim 6, wherein the gas(es) supplied to the chamber are process gas(es) from which the plasma is generated.

8. A method according to claim 6 or claim 7, wherein the gas removed from the chamber comprises the volatile products.

9. A method according to any of claims 6 to 8, wherein substantially constant plasma conditions are maintained within the chamber during exposure of the object.

## Patentansprüche

1. Verfahren zum Sterilisieren eines Gegenstandes, welcher organisches Material trägt, wobei das Verfahren umfasst:

   Aussetzen des Gegenstandes einem Plasma eines ionisierten Gases und gleichzeitiges Aussetzen eines Kontrollgegenstandes (20) dem gleichen Plasma, wobei der Kontrollgegenstand ein Substrat umfasst, welches eine Substanz trägt,

   wobei das organische Material auf dem Gegenstand und die Substanz auf dem Substrat mit dem Plasma reagieren, um ein oder mehrere flüchtige Produkte zu erzeugen, solchermaßen, dass die Reaktion eine sichtbare Änderung des Kontrollgegenstandes bewirkt, indem die Substanz von dem Substrat entfernt wird;
   Entfernen des (der) Produkts (Produkte); und

Überprüfen des Kontrollgegenstandes, um zu bestimmen, ob das Aussetzen dem Plasma aufgetreten ist.

2. Verfahren nach Anspruch 1, wobei das organische Material Prionen umfasst.

3. Verfahren nach Anspruch 1 oder 2. wobei das Plasma ein auf Sauerstoff- oder Fluorgas basierendes Plasma ist.

4. Verfahren nach einem der Ansprüche 1 bis 3, wobei die Substanz ein Photoresist umfasst.

5. Verfahren nach einem der Ansprüche 1 bis 4, wobei die Substanz opak ist, das Substrat, welches die Markierung trägt, von der Substanz bedeckt ist und während der folgenden Reaktion der Substanz mit dem Plasma aufgedeckt wird.

6. Verfahren nach einem der vorangehenden Ansprüche, wobei das Plasma innerhalb einer Kammer (2) erzeugt wird und wobei während der Aussetzung des Gegenstandes dem Plasma, ein oder zwei Gase der Kammer zugeführt und von dieser entfernt wird bzw. werden, solchermaßen, dass ein im wesentlichen konstanter Druck innerhalb der Kammer beibehalten wird.

7. Verfahren nach Anspruch 6, wobei das (die) Gas (Gase), welche der Kammer zugeführt werden, ein Verfahrens- bzw. Prozessgas (Verfahrens- bzw. Prozessgase) ist (sind), aus welchem (welchen) das Plasma erzeugt wird.

8. Verfahren nach Anspruch 6 oder 7, wobei das aus der Kammer entfemte Gas die flüchtigen Bestandteile umfasst.

9. Verfahren nach einem der Ansprüche 6 bis 8, wobei die im wesentlichen konstanten Plasmabedingungen innerhalb der Kammer während der Aussetzung des Gegenstandes beibehalten werden.

## Revendications

1. Procédé de stérilisation d'un objet comportant une matière organique, le procédé consistant à :

exposer l'objet à un plasma de gaz ionisé et simultanément exposer un objet de contrôle (20) au même plasma, l'objet de contrôle comprenant un substrat comportant une substance, moyennant quoi la matière organique sur l'objet et la substance sur le substrat réagissent chacun avec le plasma pour générer un ou plusieurs produits volatils de sorte que la réaction provoque un changement visible dans l'objet de contrôle par le retrait de la substance du substrat ;
retirer le(s) produit(s) ; et
contrôler l'objet de contrôle pour déterminer si l'exposition au plasma a eu lieu.

2. Procédé selon la revendication 1, dans lequel la matière organique comprend des prions.

3. Procédé selon la revendication 1 ou la revendication 2, dans lequel le plasma est un plasma à base de gaz oxygène ou fluor.

4. Procédé selon l'une quelconque des revendications 1 à 3, dans lequel la substance comprend une résine photosensible (21).

5. Procédé selon l'une quelconque des revendications 1 à 4, dans lequel la substance est opaque, le substrat comportant des indices recouverts par le substrat et qui sont découverts suite à la réaction de la substance avec le plasma.

6. Procédé selon l'une quelconque des revendications précédentes, dans lequel le plasma est généré à l'intérieur d'une chambre (2) et, pendant l'exposition de l'objet au plasma, un ou plusieurs gaz sont introduits dans et retirés de la chambre de sorte qu'une pression sensiblement constante est maintenue à l'intérieur de la chambre.

7. Procédé selon la revendication 6, dans lequel le(s) gaz introduits) dans la chambre est (sont) un (des) gaz de traitement à partir duquel (desquels) le plasma est généré.

8. Procédé selon la revendication 6 ou la revendication 7, dans lequel le gaz retiré de la chambre comprend les produits volatils.

9. Procédé selon l'une quelconque des revendications 6 à 8, dans lequel des conditions de plasma sensiblement constantes sont maintenues à l'intérieur de la chambre pendant l'exposition de l'objet.

# Fig.1.

Process gas 1
Vent (nitrogen)
Process gas 2
Process gas 3

Gas pod
25

Barrel (chamber)

Door

8

14

Plasma

4

Plasma

6

6A

5

10

2

1

3

11

27

Earth (ground)

Radio frequency (RF) power generator

Cable

26

10 Torr cm gauge

28

Vacuum interlock switch

Automatch unit

29

Isolation valve (pneumatically actuated)

12

13

Throttle valve (manually or electronically controlled)

Pump

9

EP 1 381 400 B1

# Fig.2.

21
20

# Fig.3.

Complete ◄——— 22

# Fig.4.

| Load chamber | ~100 |
| Evacuate chamber | ~101 |
| Introduce gas (es) | ~102 |
| Generate plasma | ~103 |
| Finish | ~104 |
| Monitor | ~105 |